# EUROPEAN PATENT SPECIFICATION

(11) **EP 2 442 779 B1**
(45) Date of publication and mention of the grant of the patent: **06.11.2013**
(21) Application number: 10723075.7
(22) Date of filing: 20.05.2010
(51) Int. Cl.: A61K 8/02, A61K 8/06, A61K 8/97, A61Q 15/00

(54) **ANTIPERSPIRANT USAGE**
SCHWEISSHEMMERVERWENDUNG
Utilisation anti-transpirante

(30) Priority: 16.06.2009 EP 09162778
(43) Date of publication of application: 25.04.2012
(73) Proprietor: Unilever PLC, A Company Registered in England and Wales under Company no. 41424, London, Greater London EC4Y 0DY (GB); Unilever NV, 3013 AL Rotterdam (NL)
(72) Inventor: COURTOIS, Jean-Philippe, Andre, Roger, Wirral Merseyside CH63 3JW (GB); HARDING, Clive, Roderick, Wirral Merseyside CH63 3JW (GB); HARKER, Mark, Wirral Merseyside CH63 3JW (GB); KELSO, Hailey, Wirral Merseyside CH63 3JW (GB); WEDDELL, Iain, Andrew, Wirral Merseyside CH63 3JW (GB)
(74) Representative: Whaley, Christopher
(86) International application number: PCT/EP2010/056985
(87) International publication number: WO 2010/145909

(56) References cited:
- WO-A-00/47182
- WO-A-2006/110753
- WO-A-2009/063016
- WO-A1-2007/071474
- DE-A1- 10 057 767

## Description

This invention relates to the field of cosmetic products, in particular antiperspirant products and usage. This invention also relates to methods of reducing perspiration upon the surface of the human body.

Cosmetic compositions used for reducing perspiration often comprise an astringent metal salt, such as aluminium or zirconium salt. Such salts are perceived as harsh or irritating by some consumers and there is a need for alternative means of achieving sweat reduction.

The present invention utilises lamellar phase stabilised oil-in-water emulsions as antiperspirant agents. Such agents have been widely used in cosmetic compositions; however, their antiperspirant properties have not been previously recognised.

In a first aspect of the present invention, there is provided the use of a lamellar phase stabilised oil-in-water emulsion as an antiperspirant agent or sweat reducing agent.

In a second aspect of the present invention, there is provided the use of a composition comprising lamellar phase stabilised oil-in-water emulsion and excluding any astringent aluminium or zirconium metal salt as an antiperspirant.

The methods and uses of the present invention should be understood to be non-therapeutic in nature, typically achieved by the topical application of one or more cosmetic compositions. The methods and uses may be termed "cosmetic". The methods lead to reduction in perspiration or sweating, which can enhance an individual's comfort, appearance, and/or confidence.

The lamellar phase stabilised oil-in-water emulsion may be used to give antiperspirancy in the absence of any other agent capable of giving this benefit. Alternatively, the lamellar phase stabilised oil-in-water emulsion may be used with another antiperspirant agent.

When the lamellar phase stabilised oil-in-water emulsion is used in conjunction with a further antiperspirant agent, it is not essential that the two are part of the same composition. Independent application of the two materials may be employed. Such application may be concurrent or consecutive, provided that the treated surface experiences the presence of both materials. When the actives are applied from independent compositions, it is preferred that the product also comprises a means for, and/or instruction for, both of the compositions to be applied to the surface requiring treatment.

Lamellar phase stabilised oil-in-water emulsions as used in the present invention may be called oleosome dispersions. Typically, they comprise dispersed droplets of oil surrounded by multiple layers of liquid crystalline lamellar phase comprised of one or more surfactants, non-ionic surfactants being preferred.

The average particle size of the lamellar phase stabilised oil-in-water emulsion is preferably at least 4 microns. Such measurements may be made using optical microscopy. Using oleosome dispersions of lower average particle size can lead to reduced performance.

The lamellar phase stabilised oil-in-water emulsion typically forms part of a cosmetic composition. Further features of such compositions are described in the following paragraphs.

The oil of the oleosome dispersions should preferably remain in a liquid state at temperatures as low as 20°C or even 15°C. For this reasons, typical oils have melting point from their solid state of less than 20°C, preferably less than 15°C, and more preferably less than 10°C.

Preferred oils should be relatively fluid, having a kinematic viscosity of less than 100 cSt (mm²/s) and preferably less than 50 cSt (mm²/s) at 20°C.

Suitable oils include hydrocarbon oils and ester oils, particularly triglyceride oils, such as sunflower seed oil. Other particular oils that might be used in the oleosomes are C₁₂₋₁₅ alkyl benzoate esters, hydrogenated polybutene, PPG-14 butyl ether, triethyl citrate, isopropyl palmitate, and isopropylmyristate.

The lamellar phase of the oleosome dispersion is typically comprised of two non-ionic surfactants, one having a relatively low HLB (less than 8 and preferably less than 5) and one having a relatively high HLB (more than 12 and preferably more than 15). The ratio high HLB surfactant to low HLB surfactant is chosen so as to give a stable oleosome dispersion, typically ratios being from 1:1 to 1:6 by weight, respectively. A blend of steareth-2 and steareth-20 has been found to be suitable, particularly when used in combination with sunflower oil. Steareth-2 and steareth-20 are best used at a ratio of from 1:4 to 1:5 by weight.

The oleosome dispersions have an oil content that typically makes up from 1 to 30%, preferably from 1 to 20%, and more preferably from 1 to 10% by weight of the total composition. The surfactant content typically makes up from 1 to 30%, preferably from 1 to 20%, and more preferably from 1 to 10% by weight of the composition. The ratio of oil to surfactant is typically from 1:3 to 3:1 and preferably from 1:2 to 2:1.

The method of controlling perspiration offered by the invention is particularly useful because the benefit can extend for a considerable period of time, often greater than 24 hours and sometimes even up to 4 days after application or longer. Significant deodorancy benefits may also accrue from the use of the present invention.

The method of controlling perspiration is particularly useful when direct application to the surface of the human body is involved. This is especially true when application to the axillae and/or feet is involved. Application to the axillae is most preferred because of the high concentration of eccrine sweat glands in these regions of the human body.

The method of controlling perspiration typically involves multiple applications of the oleosome dispersion. It is thought that multiple applications improve perspiration reduction by some form of build up effect. A composition comprising the oleosome dispersion may be applied as part of one's daily regime, after washing, for example.

In this description, the term "comprising" should be understood to be non-exhaustive, i.e., meaning that other components may also be present.

An anti-microbial deodorant agent is a preferred additional component of compositions suitable for use in accordance with the present invention. The anti-microbial deodorant agent may be a bacteriocidal agent or a bacteriostatic agent. Synergistic deodorancy benefits may be achieved using compositions comprising an anti-microbial deodorant agent.

Preferred anti-microbial deodorant agents are organic in nature and such anti-microbial deodorant agents are particularly preferred in compositions that do not comprise an astringent salt of aluminium and/or zirconium.

When employed, the anti-microbial deodorant agent is typically incorporated into the composition at from 0.01% to 3% and particularly at from 0.03% to 0.5%.

Preferred anti-microbial deodorant agents have a minimum inhibitory concentration (MIC) of 1 mg.ml⁻¹ or less, particularly 200 µg.m⁻¹ or less, and especially 100 µg.ml⁻¹ or less. The MIC of an anti-microbial agent is the minimum concentration of the agent required to significantly inhibit microbial growth. Inhibition is considered "significant" if an 80% or greater reduction in the growth of an inoculum of *Staphylococcus epidermidis* is observed, relative to a control medium without an anti-microbial agent, over a period of 16 to 24 hours at 37°C. Details of suitable methods for determining MICs can be found in "Antimicrobial Agents and Susceptibility Testing", C.Thornsberry, (in "Manual of Clinical Microbiology", 5th Edition, Ed. A. Balows et al, American Society for Microbiology, Washington D.C., 1991). A particularly suitable method is the Macrobroth Dilution Method as described in Chapter 110 of above publication (pp. 1101-1111) by D. F. Sahm and J. A. Washington II. MICs of anti-microbials suitable for inclusion in the compositions of the invention are triclosan: 0.01-10 µg.ml-1 (J.Regos et al., Dermatologica (1979), 158: 72-79) and farnesol: ca. 25 µg.ml⁻¹ (K. Sawano, T. Sato, and R. Hattori, Proceedings of the 1th IFSCC International Conference, Yokahama (1992) p.210-232). By contrast ethanol and similar alkanols have MICs of greater than 1 mg.ml⁻¹.

Suitable organic anti-microbials are bactericides, for example quaternary ammonium compounds, like cetyltrimethylammonium salts; chlorhexidine and salts thereof; and diglycerol monocaprate, diglycerol monolaurate, glycerol monolaurate, and similar materials, as described in "Deodorant Ingredients", S.A.Makin and M.R.Lowry, in "Antiperspirants and Deodorants", Ed. K. Laden (1999, Marcel Dekker, New York). More preferred anti-microbials for use in the compositions of the invention are polyhexamethylene biguanide salts (also known as polyaminopropyl biguanide salts), an example being Cosmocil CQ™ available from Zeneca PLC, preferably used at up to 1% and more preferably at 0.03% to 0.3% by weight; 2',4,4'-trichloro,2-hydroxy-diphenyl ether (triclosan), preferably used at up to 1% by weight of the composition and more preferably at 0.05-0.3%; and 3,7,11-trimethyldodeca-2,6,10-trienol (farnesol), preferably used at up to 1% by weight of the composition and more preferably at up to 0.5%.

Other suitable organic antimicrobial agents are transition metal chelators, as described in WO01/52805, for example. Transitional metal chelators having a binding coefficient for iron(III) of greater than 10²⁶, for example diethylenetriaminepentaacetic acid and salts thereof are preferred.

The lamellar phase stabilised oil-in-water emulsion may be applied to the surface of the human body by any means. Liquid compositions comprising the lamellar phase stabilised oil-in-water emulsion can be by absorption onto a carrier matrix like paper, fabric, or sponge and applied by contacting said carrier matrix with the surface. Application can also comprise a combination of any two or more of the above techniques.

Other cosmetically acceptable components may also be present in compositions used in accordance with the present invention. Such will vary according to the desired mode of application of the composition. For example, a volatile propellant is a typical component in compositions for spray application and a liquid carrier fluid (usually water) is a typical component in compositions for roll-on application. Thickeners, in particular cellulosic thickeners such as hydroxypropylcellulose and hydroxyethylcellulose may also be used in roll-on compositions.

Certain sensory modifiers are further desirable components. Such materials are preferably used at a level of up to 20% by weight of the composition. Emollients, humectants, volatile oils, non-volatile oils, and particulate solids which impart lubricity are all suitable classes of sensory modifiers. Examples of such materials include cyclomethicone, dimethicone, dimethiconol, isopropyl myristate, isopropyl palmitate, talc, finely-divided silica (eg. Aerosil 200), particulate polyethylene (eg. Acumist B18), polysaccharides, corn starch, C12-C15 alcohol benzoate, PPG-3 myristyl ether, octyl dodecanol, C7-C14 isoparaffins, di-isopropyl adipate, isosorbide laurate, PPG-14 butyl ether, glycerol, hydrogenated polyisobutene, polydecene, titanium dioxide, phenyl trimethicone, dioctyl adipate, and hexamethyl disiloxane.

Fragrance is also a desirable additional component. Suitable materials include conventional perfumes, such as perfume oils and also include so-called deoperfumes, as described in EP 545,556 and other publications. Levels of incorporation are preferably up to 4% by weight, particularly from 0.1 % to 2% by weight, and especially from 0.7% to 1.7% by weight.

It should be noted that certain components of compositions perform more than one function. Such components are particularly preferred additional ingredients, their use often saving both money and formulation space. Examples of such components include ethanol, isopropyl myristate, and the many components that can act as sensory modifiers.

Further additional components that may also be included are colourants and preservatives at a conventional concentration, for example C₁-C₃ alkyl parabens.

### Examples

In the following examples all percentages are by weight, unless otherwise indicated.

The composition indicated in Table 1 was prepared using methods known in the art. The steareth-2, steareth-20, and sunflower oil formed a lamellar phase stabilised oil-in-water emulsion.

**Table 1**

| **Chemical Name** | **Amount (%)** | **Trade Name** | **Supplier** |
|---|---|---|---|
| | **Example 1** | | |
| Water and minors | 92.8 | | Local |
| Helianthus Annuus* | 4.0 | Agri Pure 80 | Cargill |
| Steareth-2 | 2.6 | Volpo S2 | Croda |
| Steareth-20 | 0.6 | Brij 78 | Uniqema |

| | | | |
|---|---|---|---|
| * Sunflower seed oil. | | | |

The following protocol was used to evaluate the compositions of Table 1. Trained operators applied the test composition to a first axilla of panellists once each day for four consecutive days in week one and then for three consecutive days in week two (ca. 0.3g per axilla). A control composition was applied to the panellists' second axilla. The panellists self-applied at home at the weekend in between the two test weeks. A conventional roll-ball applicator was used for the applications. At intervals during the test, panellists were required to sit in a hot room (temperature: 40°C; relative humidity: 40%) for up to one hour and twenty minutes. During the sittings, sweat was collected on absorbent cotton pads held in each axilla and the amount produced was recorded.

Hot room sittings were done after the periods indicated in Table 2. The final sitting was done 96 hours after the final applications, the panellists being allowed to wash as normal during the intervening period. Table 2 indicates the "Sweat Weight Reduction (SWR)" achieved by the test composition relative to the control composition (which was a simple ethanolic deodorant, lacking any antiperspirant active).

**Table 2**

| **Time*** | **SWR (%)** |
|---|---|
| 4 days | 11.4 |
| 5 days | 17.7 |
| 10 days | 22.9 |
| + 4 days "wash out" | 9.4 |

| | |
|---|---|
| * Time after initial application. Hot room sittings done before application on days 4 and 7. | |

Significant SWRs were achieved with Composition 1, even after the 4 day "wash out" period.

The compositions indicated in Table 3 may be prepared by methods known in the art. Compositions comprising both lamellar phase stabilised oil-in-water emulsion and an organic anti-microbial, can deliver both significant antiperspirancy and significant deodorancy.

**Table 3**

| **Component** | **Amount (%)** | | | | |
|---|---|---|---|---|---|
| **Composition:** | **2** | **3** | **4** | **5** | **6** |
| Water (+ preservatives¹) | 92.7 | 92.4 | 92.7 | 92.8 | 87.5 |
| Sunflower oil | 4.0 | -- | 4.0 | -- | 4.0 |
| Isopropyl myristate | -- | 4.0 | -- | -- | -- |
| C₁₂₋₁₅ alkyl benzoate ester | -- | -- | -- | 4.0 | -- |
| Steareth-2 | 2.6 | 2.8 | 2.6 | 2.6 | 2.6 |
| Steareth-20 | 0.6 | 0.7 | 0.6 | 0.6 | 0.6 |
| Glycerol | -- | -- | -- | -- | 4.9 |
| Triclosan¹ | 0.1 | -- | -- | -- | -- |
| Cosmocil CQ² | - | 0.1 | 0.1 | -- | -- |
| 2-Phenoxyethanol | - | -- | -- | -- | 0.4 |

| | | | | | |
|---|---|---|---|---|---|
| 1. 2',4,4'-trichloro,2-hydroxy-diphenyl ether. 2. Polyhexamethylene biguanide salt. | | | | | |

Examples 5 and 6 were evaluated by a method analogous to that described for the composition of Table 1. The results are shown in Table 4 and the differences to the control were each significant at the 95% level.

**Table 4**

| **Time** | **SWR (%)** | |
|---|---|---|
| **Example:** | **5** | **6** |
| 4 days | 13.9 | 13.4 |
| 7 days | -- | 26.7 |
| 10 days | -- | 26.1 |
| + 5 days "wash out" | -- | 19.8 |

## Claims

1. The use of a lamellar phase stabilised oil-in-water emulsion as an antiperspirant agent.

2. The use according to claim 1, wherein the lamellar phase stabilised oil-in-water emulsion comprises oil droplets surrounded by multiple layers of liquid crystalline phase comprised of one or more surfactants.

3. The use according to claim 2, wherein the multiple layers of liquid crystalline phase comprise non-ionic surfactant.

4. The use according to claim 3, wherein the multiple layers of liquid crystalline phase comprise two non-ionic surfactants, one having a HLB of less than 5 and another having a HLB of greater than 15.

5. The use according to any of preceding claims, wherein the emulsified oil has a melting point from its solid state of less than 15°C.

6. The use according to any of preceding claims, wherein the emulsified oil has a kinematic viscosity of less than 50 mm²/s at 20°C

7. The use according to any of preceding claims, wherein the emulsified oil is a triglyceride oil.

8. The use according to any of preceding claims, comprising multiple applications of the lamellar phase stabilised oil-in-water emulsion.

9. The use of an oleosome dispersion for antiperspirancy or sweat reduction.

10. The use of a composition comprising lamellar phase stabilised oil-in-water emulsion and excluding any astringent aluminium or zirconium metal salt as an antiperspirant or sweat reducing composition.

11. A method of reducing perspiration upon the surface of the human body comprising the application of a composition comprising lamellar phase stabilised oil-in-water emulsion and excluding any astringent aluminium or zirconium metal salt.

## Patentansprüche

1. Verwendung einer durch lamellare Phase stabilisierten Öl-in-Wasser-Emulsion als schweißhemmendes Mittel.

2. Verwendung gemäß Anspruch 1, wobei die durch lamellare Phase stabilisierte Öl-in-Wasser-Emulsion Öltropfchen umfasst, die von mehreren Schichten flüssigkristalliner Phase, umfassend ein oder mehrere Tensid(en), umgeben sind.

3. Verwendung gemäß Anspruch 2, wobei die mehreren Schichten flüssigkristalliner Phase nicht-ionisches Tensid umfassen.

4. Verwendung gemäß Anspruch 3, wobei die mehreren Schichten flüssigkristalliner Phase zwei nicht-ionische Tenside umfassen, wobei eines einen HLB-Wert von weniger als 5 hat und ein anderes einen HLB-Wert von größer als 15 hat.

5. Verwendung gemäß einem der vorangehenden Ansprüche, wobei das emulgierte Öl einen Schmelzpunkt aus seinem festen Zustand von weniger als 15°C hat.

6. Verwendung gemäß einem der vorangehenden Ansprüche, wobei das emulgierte Öl eine kinematische Viskosität bei 20 °C von weniger als 50 mm2/s hat.

7. Verwendung gemäß einem der vorangehenden Ansprüche, wobei das emulgierte Öl ein Triglyceridöl ist.

8. Verwendung gemäß einem der vorangehenden Ansprüche, die mehrfache Anwendungen der durch lamellare Phase stabilisierten Öl-in-Wasser-Emulsion umfasst.

9. Verwendung einer Oleosom-Dispersion zur Schweißhemmung oder Schweißverringerung.

10. Verwendung einer Zusammensetzung, die eine durch lamellare Phase stabilisierte Öl-in-Wasser-Emulsion umfasst und ein adstringentes Aluminium- oder Zirkoniummetallsalz ausschließt, als schweißhemmende oder schweißverringernde Zusammensetzung.

11. Verfahren zur Verringerung des Schwitzens an der Oberfläche des menschlichen Körpers, umfassend die Anwendung einer Zusammensetzung, welche eine durch lamellare Phase stabilisierte Öl-in-Wasser-Emulsion umfasst und ein adstringentes Aluminium- oder Zirkoniummetallsalz ausschließt.

## Revendications

1. Utilisation d'une émulsion huile dans eau stabilisée par une phase lamellaire en tant qu'agent anti-transpirant.

2. Utilisation selon la revendication 1, dans laquelle l'émulsion huile dans eau stabilisée par une phase lamellaire comprend des gouttelettes d'huile entourées par de multiples couches de phase cristalline liquide comprenant d'un ou plusieurs tensioactifs.

3. Utilisation selon la revendication 2, dans laquelle les multiples couches de phase cristalline liquide comprennent un tensioactif non ionique.

4. Utilisation selon la revendication 3, dans laquelle les multiples couches de phase cristalline liquide comprennent deux tensioactifs non ioniques, un ayant un HLB inférieur à 5 et l'autre ayant un HLB supérieur à 15.

5. Utilisation selon l'une quelconque des revendications précédentes, dans laquelle l'huile émulsifiée a un point de fusion à partir de son état solide inférieur à 15 °C.

6. Utilisation selon l'une quelconque des revendications précédentes, dans laquelle l'huile émulsifiée a une viscosité cinématique inférieure à 50 mm²/s à 20 °C.

7. Utilisation selon l'une quelconque des revendications précédentes, dans laquelle l'huile émulsifiée est une huile de triglycéride.

8. Utilisation selon l'une quelconque des revendications précédentes, comprenant de multiples applications de l'émulsion huile dans l'eau stabilisée par une phase lamellaire.

9. Utilisation d'une dispersion d'oléosome pour un effet anti-transpirant ou une réduction de la transpiration.

10. Utilisation d'une composition comprenant une émulsion huile dans l'eau stabilisée par une phase lamellaire et excluant tout sel métallique d'aluminium ou de zirconium astringent en tant que composition anti-transpirante ou de réduction de la transpiration.

11. Procédé de réduction de la transpiration sur la surface du corps humain comprenant l'application d'une composition comprenant une émulsion huile dans l'eau stabilisée par une phase lamellaire et excluant tout sel métallique d'aluminium ou de zirconium astringent.
